Europäisches Patentamt

European Patent Office

Office européen des brevets

(11) Publication number: **0 388 486**
**A1**

## EUROPEAN PATENT APPLICATION

(21) Application number: **89104959.5**

(22) Date of filing: **20.03.89**

(51) Int. Cl.⁵: **A61M 29/02**

(43) Date of publication of application:
**26.09.90 Bulletin 90/39**

(84) Designated Contracting States:
**AT BE CH DE ES FR GB GR IT LI LU NL SE**

(71) Applicant: **Dick, Gianfederico**
**Via Bellini 20**
**I-20052 Monza (Province of Milano)(IT)**

(72) Inventor: **Dick, Gianfederico**
**Via Bellini 20**
**I-20052 Monza (Province of Milano)(IT)**

(54) Steerable "PTCA" dilatation catheter with a regulating expansion section.

(57) Steerable percutaneous transluminal coronary angioplasty (PTCA) dilatation catheter with a regulating expanding device (10) embodied in a connector (9) having three ports: One for pressure application (7) at the pre-set expandable section (3); one for pressure, time and temperature reading (8) of the fluid at the same expandable section, and one for introducing guide wire (10). The interlumen of the longitudinal circular body is made of a polymer coated metal coil tightly wound (5) and an outer jacket (4) with one single alternating section of closed and open braided mesh of unstretchable material of ultra high modulus metals or fibers. At the distal end of the catheter the two bodies are sealed at (2) to form the passageway (53) for the fluid. The steerable PTCA catheter with the expandable section can be internally pressurized with or without inflation of the expandable section, the degree of expansion is controlled by the regulating device.

FIG.6

## STEERABLE "PTCA" DILATATION CATHETER WITH A REGULATING EXPANSION SECTION.

The present invention relates to a medical device to be used in dilatation of blood vessels in human bodies which are affected by stenosis, i.e. narrow strictures, more particularly, to a steerable percutaneous transluminal coronary angioplasty PTCA dilatation catheter with an embodied expandable section called balloon, which diameter can be regulated according to the patient arteries size by the operating physician so as to reduce overdilatation and higher risk of restenosis formation and bypass surgery. This steerable PTCA dilatation catheter can be internally pressurized without balloon formation. This feature is embodied to help the physician to pass narrow and hard stricture in the blood vessel.

The historical background of dilatation started already in the sixties by Dottier and Judkins who performed the first intraluminal angioplasty using a coaxial double catheter system to stretch the lumen of femoral arteries. Porstmann and Zeitlar utilized a larged distensible balloon for peripheral arteries dilatation. The technique was then modified by A. R. Gruentzig who developed a cylindrical balloon for use in PTA peripheral arterial angioplasty and further modified to the PTCA technique. In coronary angioplasty, also called "PTCA", percutaneous transluminal coronary angioplasty, the balloon dilatation system has been well established although with some concern with the existing equipment limited possibilities and devices of different characteristics. It has not yet been fully established the degree of the dilatation of the arteries which will prevent reformation of stenosis, i.e. or reduce its rate, overstretching could be one of the reasons, that is to say the more lesions dilated, the higher the risk of restenosis recurrence, Restenosis Coronary Angioplasty, B. Meier p. 161, 1987; since approximately 30% of the patients develop restenosis within 6 months following coronary angioplasty, Libow, I Am. Gel. Cardiol 5: 45, 1985. Recurrent stenosis has not been well studied pathologically. The pathology of this recurrence and the methods to prevent it remain the most important aspect of angioplasty research of today. Spencer B. King, PTCA chap.17, pp 457, 1987.

One of the most recent and prominent publications as to the problem involved with overdilatation in PTCA is given by the world famous experts on the field. Part of the abstract is here quoted in full form;

"Restenosis after percutaneous transluminal coronary angioplasty (PTCA) is strongly associated with incomplete initial dilatation. To determine if oversized PTCA balloons would reduce the restenosis rate without increasing the risk of arterial dissection and acute complications, we prospectively randomized 336 patients to receive either smaller or larger balloons.

We conclude that satisfactory initial results can be achieved by conservative sizing of balloon catheters and attention to other dilatation details such as inflation pressure and times. The intention to reduce restenosis by oversizing balloons will result in increased complications, particularly in patients with multivessel disease or complex lesion morphology. (Circulation 1988; 78: 557-565)."

There is no doubt about the advantages obtained as to blood vessel dilatation concern, when the operating physician has the possibilities of performing a well controlled dilatation degree of the arteries in conformity with the patient artery size. Accordingly there is a need of a dilatation catheter which has a very limited translational shear force at the wall of the blood vessel during balloon inflation combined with the outward appearance of tubing of consistent diameter over its full length with an adjustable balloon diameter which can be internally pressurized at equal pressure, but at different balloon sizes. The object of the present invention is to provide such dilatation catheter of small diameter to be mainly used in coronary angioplasty.

Generally speaking, the dilatation catheter of today usually comprises a tube with plural or single channel where to one end a balloon is attached for inflation purposes. The main parts of the catheter are commonly called "shaft" and the passage inside the "lumen". The catheter beside the tube, i.e. shaft, comprises a connection device with plural or single port.

The commonly used terminology in describing a dilatation catheter in general terms, one refers to a coaxial and non-coaxial inflation system. The coaxial system is normally a two lumen catheter where in the inner lumen of the shaft a second tube is inserted and attached at the distal end, thus forming a passage for the inflation of a "balloon" through a "y" connector system where the shaft assembly is connected. The non-coaxial system is mainly constituted of a shaft with one or more internal lumens where at the distal end a preformed plastic non-woven balloon is attached. The former type of catheter has a woven balloon attached to a plastic tube, and the working system is in general the following: When the catheter is internally pressurized, the inner tube in the shaft moves against a compensating device embodied in the connector thus to accomodate the decrease in the length of the woven balloon when under pressure. Upon release of the applied pressure the compensating device returns the inner tube to its distal position and the balloon diameter is seldom reduced to its former diameter. Hanecka, Olbert, GB 1.566.674.

A modified system of the coaxial principle is the stretching expanding movement of the shaft. This system avoids the use of compensating devices in the connector for retraction movements during the inflation operation. The working system is as follows:

Upon release of the applied pressure at the stretching expanding section the two translational movements will work in opposite directions during deflation and inflation. The former diameter of the balloon of the catheter is approximately regained, US patent 4.706.670.

The non-coaxial system principle is mainly based on radial expansion movement of the balloon during pressure application. The pressurized fluid passes through a lumen and then reaches the preformed baloon, thus inflating it. The retraction mechanism in the system is not present, and the deflated diameter can only be achieved by aspiration of the injected fluid, US patent 4.195.637.

The shortcomings of the coaxial system are undoubtedly the inconsistency of keeping with time at body temperature a constant balloon diameter when under constant working pressure and above 14 atm. and the shearing effect at the blood vessel. Both the modified and the non-modified coaxial system are bound to have translational motion during the application of pressure and limited recovery of the balloon diameter after deflation. These catheter types ae today only present in the radiology field. Further drawbacks of the non-coaxial and coaxial system are the lack of providing a balloon catheter which has the outward appearance of tubing of consistent diameter over its full length, and limited internal balloon working pressure 10-14 atm. All three principles lack of adjustable balloon diameters and incapability to work with different controllable baloon diameter sizes at equal internal fluid pressure. The coaxial principle is not yet seen in the PTCA procedure, i.e. cardiology.

The dilatation catheter of the present invention is in principle a hybrid system which is intended to remedy the drawbacks of the two main systems and fulfill the latest demands of the physician in cardiology. The outer shaft is a flexible tubing reinforced by non-stretchable material of extremely low elongation under stress application. Contiguous to the flexible reinforced tubing an elastomeric material makes up the "pre-set diameter section" which is also reinforced by the same unstretchable material forming the expansion section. The inner section generally called the inner-tube is made of a metallic coil forming an incompressible flexible spring which is outside jacketed by a polymeric flexible material. The distal end of the pre-set diameter section is anchored to the jacket spring thus forming passageway for inflation. The metallic incompressible spring proximal end is anchored at an adjustable length device in one of the connector ports. The outer shaft is built like a composite where the inner and outer layer are made of elastomeric materials which enclose a reinforcing material of high modulus of elasticity like metals, steel, carbon, glass, fibers, boron, ceramic, aramid, wiskers or other polymeric materials of very low elongation at break, less than 4% like Kevler (Du Pont trademark). The reinforcing material is tightly braided for most of the total length of the tubular body, the remaining part is reinforced with a more open braiding, thus resulting in an expandable section, so-called balloon. When the working pressure is applied at the connector port by a syringe filled with contrast media, there will be no expansion of the pre-set expansion section, i.e. no balloon formation unless the spring was adjusted to a chosen diameter value in the connector diameter regulating device.

Before any inflation of the balloon is made, the physician has the possibilities of adjusting the regulating device to a position which will give a pre-set balloon diameter during inflation. The pre-set section becomes shorter by pulling backwards the spring with the aid of the regulating device turning knob. In the knob an arrow will indicate the minimum and maximum achievable balloon size and with minimum wall shear forces at the intima during balloon inflation.

The application of the internal pressure to the catheter interlumen has an opposite balance effect. The pre-set length of the spring at the regulating device, i.e. shortening of the expansion section, prevents the balloon to achieve a maximum diameter under working pressure due to the incompressible spring attached to the connector regulating device. The catheter is designed to work with different expansion levels at the same internal pressure. Under pressure application the balloon can only reach a diameter that was pre-set by the operator before inflation. Even if there is an increase in pressure at the inflation port, the balloon will not increase its diameter. When pressure is released at the connector port, the balloon will regain its pre-set deflated diameter value. Consequently, a new desired balloon diameter can be achieved during the dilatation procedure by the regulating expansion, with or without unpressurizing the system. The very fine regulating device in the connector allows the operating physician to avoid the use of more than one balloon catheter during the dilatation procedure. Furthermore, the present invention allows the dilatation catheter to be fit in the blood vessel stricture with a regulating expandable balloon size that satisfies the enlargement of the stenosis and minimizes vessel wall rupture due to over inflation of the balloon. This new method of dilatation gives to the operating physician a newly available and safer device for the dilatation procedure. Accordingly, it is an object of the invention to remedy the drawbacks of the coaxial and non-coaxial system for dilatation purposes in the percutaneous transluminal coronary angioplasty, and to add few more.

3

It is an object of the present invention to provide a dilatation catheter for use in PTCA which can be used without risk of over dilatation of the balloon from the desired size, and with full control of variable balloon diameters at constant pressure.

- it is still another object of the present invention to provide a balloon catheter with pre-set balloon diameter at constant working pressure.

- it is a further object of the present invention to provide a dilatation catheter which has the outward appearance of a consistent equal diameter over its full length,

- it is another object of the present invention to provide a dilatation catheter with minimum risk of balloon rupture,

- it is another object of the present invention to provide a dilatation catheter with no kinking possibilities while in use and still preserving tracking,

- it is another object of the present invention to provide a balloon catheter with adjustable torque by pressurizing without balloon inflation,

- it is another object of the present invention to provide a dilatation catheter to work at higher temperature than the body 37 degree C,

- it is another object of the present invention to provide a dilatation catheter with adjustable pushability,

- it is another object of the present invention to provide a dilatation catheter for high pressure 25 atmospheres and up to 35 in special cases,

- it is another object of the present invention to provide a dilatation catheter to have an inflated balloon visible at the video-screen without contrast media,

- it is another object of the present invention to improve the manoeuvreability of the balloon catheter by steel coil inner-body,

- it is another object of the present invention to provide a balloon dilatation catheter to be able to penetrate a narrow and hard passage due to stenosis,

- it is another object of the present invention to provide a surgical instrument which allows the physician to operate with a dilatation system in accordance with the degree of stenosis encountered in the patient,

- the present invention comprises a sophisticated manufacture processing where the properties and features of the present invention are related to each other.

The above statements are intended to describe the specified and the generic features of the present invention and all matters contained in the descriptions and shown in the attached drawings shall be understood as illustrative and not in a limiting sense.

A clear understanding of the present invention may be gained from the following detailed references and drawing description of the preferred embodiment thereof.

Fig. 1 is a plan view of the balloon catheter showing the attachments for supplying inflation fluid under pressure, regulating balloon diameter device, pressure recording port, guide wire port.

Fig. 2(a), 2(b) and 2(c) are geometric representation of the reinforced balloon section with different pre-set positions of the unstretchable material.

Fig. 3(a), 3(b) and 3(c) fragmentary vertical section of the pre-set balloon diameter before inflation, transition diameter position, final maximum inflation diameter.

Fig. 4 is a schematic perspective representation showing the adjusting thumbwheel device with the pre-set inflation possibilities.

Fig. 5 is a fragmentary and perspective section of deflated balloon catheter, tapered section and distal flexible end tip.

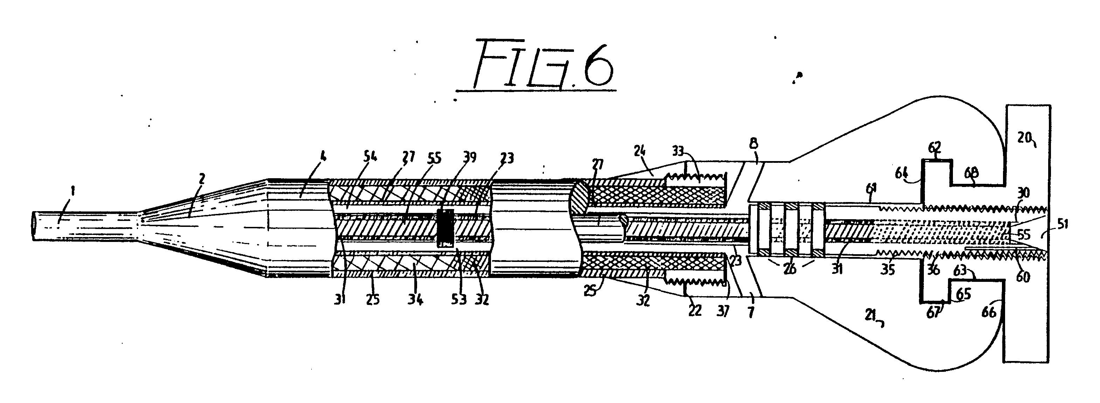

Fig. 6 is a perspective elevational view, partly broken away of the dilatation catheter showing the total assembly of the present invention.

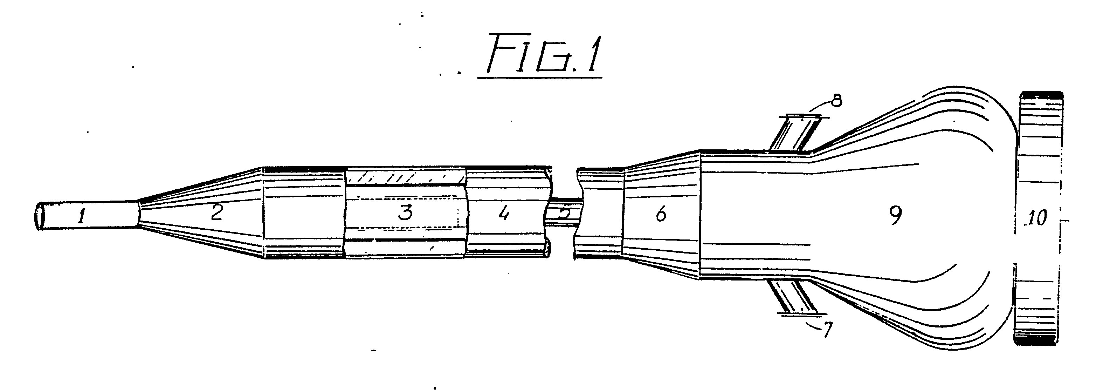

In the embodiment of the present invention illustrated in Fig. 1 the location of the regulating device 10 of the balloon diameter is embossed in the connector 9. In the connector parallel portion the one side arm 7 is used for inflation purposes, the other side arm 8 is used for pressure recording of the balloon section 3. The outer shaft 4 proximal end is embossed in the connector distal end 6. The inner-tube 5 one end is attached to the regulating device 10 of the balloon diameter; the other end distal end 2 is anchored together with the outer shaft 4. The protruding very distal end 1 forms the tip of the dilatation catheter.

The inner-tube 5 and the distal end tip 1 are continuous elements constituting the internal body of the catheter. Through passage port 7 a pressure gauge can be installed to control the inflation pressure, not shown in the drawing. The pressure of the fluid inside the balloon section 3 can be increased at a constant pre-set diameter by increasing the syringe pressure, still keeping the same balloon diameter.

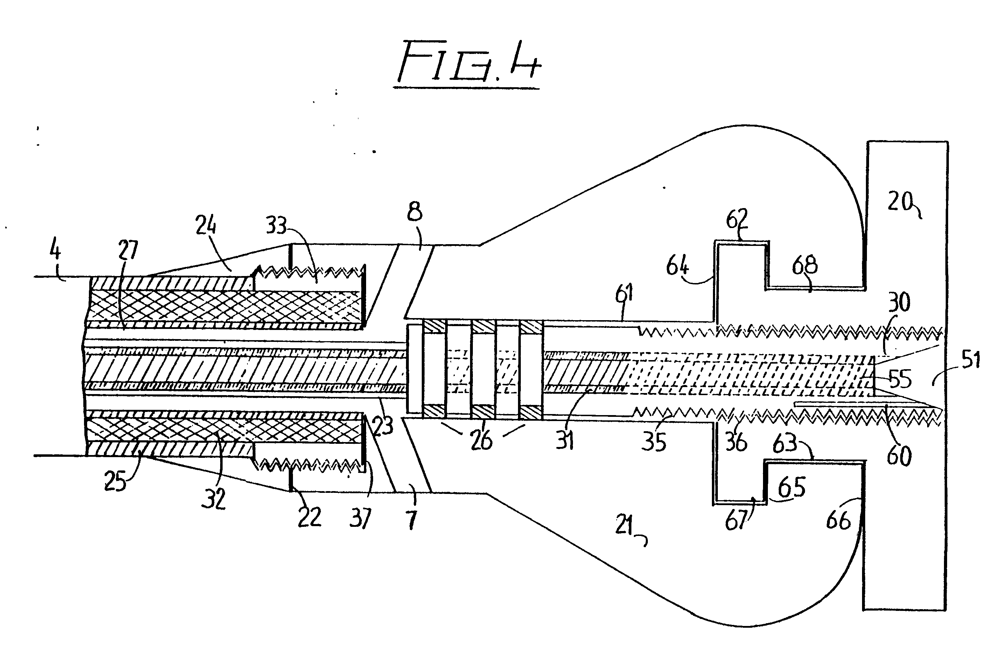

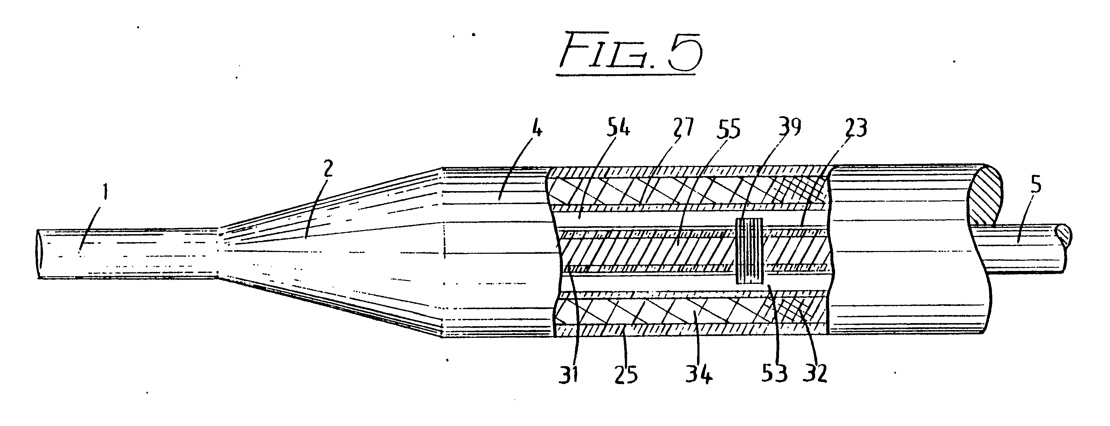

A digital timer can be inserted in 8 to display the total time of the applied pressure at the chosen balloon diameter set by the regulating device 10. The regulating balloon diameter device 10 allows to increase the balloon diameter while under pressure. The regulating balloon diameter device 10 allows to decrease the balloon diameter while under pressure without causing an increase of the pressure applied. With this arrangement the balloon catheter can be manually regulated from its proximal region to obtain the desired controlled expansion at the balloon section. This is one of the particularly innovative constructions that is embodied in the present invention shown in Fig. 4 and 5.

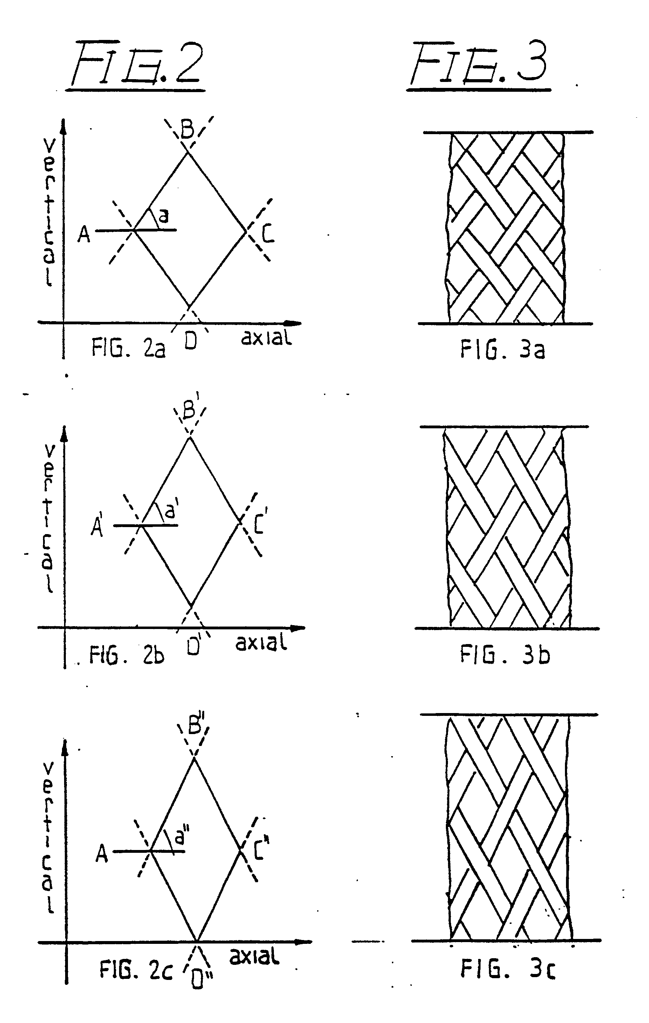

A geometrical explanation of the expanding or retracting action at the balloon site is symbolized in Fig. 2 and 3. The schematic representation in Fig. 2a and 3a is a random chosen pre-set balloon diameter size with an interval of possible values d1 < di < d2, d1 represents the lowest possible value of the balloon section which corresponds to the shaft diameter or a chosen not inflatable diameter. di is the adjustable diameter within the chosen interval (d1, d2). The relationship between Fig. 2a, 3a and Fig. 2b, 3b is explained by the fact that the pre-set value of the balloon diameter chosen before inflation is obtained by adjustment of the thumbwheel 20 in Fig. 4 at the proximal end of the conenctor 21. One of the embodiments of the invention allows the open mesh at Fig. 2a (A, B, C, D) angle (a) to be compressed in the x-axis and expanding in y-axis resulting in the configuration shown in Fig. 2b (A', B', C', D') angle (a') or 2c (A'', B'', C'', D'') angle (a'') by an axial force exerted from the thumbwheel device 20 on the distal end of the balloon section 54 in Fig. 5 which during pressure operation applied at the inflation port 7, the balloon section 54 will expand to form a cylindrical concentric longitudinal body.

In Fig. 1 the axial forces arising from injection pressure are restricted and balanced by the flexible polymer coated steel inner-coil 5 and the outer jacket 4 reinforced by unstretchable material. The inflation pressure resulting from the applied force at the syringe or mechanical device is counterbalanced by the incompression of the steel inner-coil 5 and the surrounding reinforced unstretchable section of the outer jacket 4. The only section sensitive to enlargement is the balloon section 3. A simple explicit function describing the relationship of the involved parameters can be expressed mathematically by the form:

Pbi (di, a, n, f)

The physical formula representing the burst pressure Pb for a reinforced tube is given by:

$$Pb = 2 \times n \times f \times \sin(a)/d \times 1 \quad \text{(eq. 1)}$$

$Pb$ = pressure at burst ($N/cm^2$)
$n$ = number of strands (No)
$d$ = balloon diameter (cm)
$l$ = pitch of the strand (cm)
$f$ = break force of the strand (N)
$a$ = angle of the strand with the x-axis

$$\tan(a) = pi \times di/l, \quad l = pi \times di \times \cos(a)/\sin(a) \quad \text{(eq.2)}$$

$pi = 3,1416$

$di$ = random chosen value of balloon diameter within the interval (di, d2). Combining equation (1) and (2) one obtains:

$$Pbi = 2 \times n \times f \times \sin^2(a)/pi \times di^2 \times \cos(a) \quad \text{(eq. 3)}$$

By chosing a fixed number of strands (No) and the relative breaking force (f), equation 3 can be written as:

$$Pbi = k \times \sin^2(a)/di^2 \times \cos(a) \quad \text{(eq. 4)}$$

where $k = 2 \times n \times f/pi$

di is a function of the angle (a): di(a) and $0 < a < 54.7$ degree.

Pbi will increase when: $< \sin(a)/\sqrt{\overline{\cos(a)}}$

Pbi will increase when: $di > \sin(a)/\sqrt{\overline{\cos(a)}}$

The equilibrium forces between the axial forces

$Fx = P \times D^2 \times pi/4$ and the hoop force $Fy = D \times P \times L$ is given for a reinforced cylinder by:

$$Fy = 2 Fx \quad \text{(eq. 5)}$$

which is equal to $\tan(a) = 2 \times l/d \times pi$ (eq. 6)

inserting eq. (2) $\tan(a) = d \times pi/l$ in eq. (6) and solving, one obtains the neutral angle (a) at which the two forces are balancing:

$$\tan^2(a) = 2 \Rightarrow a = 54.7 \text{ degree} \quad \text{(eq. 7)}$$

At this angle no more expansion takes place at the inflated body, when the reinforcing material has substantially no elongation at break, for instance steel. This new angle (a) is independent of the size of the inflational body; this means that all balloons at maximum size will have the reinforcement placed at an angle (a) = 54.7 degree with the x-axis.

The change in the axial forces exerted at the steel coil, due to fluid pressure applied at port 7 is derived from $dA \times dP = dF$ and eq. (1) and is here given without interim calculation:

$$F - Fo = n \times f \times \left[\sin^2(a)\right]^a_\beta \times \log \left[(1 + \sin(a)/(1 - \sin(a)))\right]^a_\beta \quad (eq. \ 8)$$

This eq. (8) expresses clearly the possibility of working at different diameters within the chosen interval d1 < di < d2 with the same balloon internal fluid pressure without risk of burst.

The applied force at the steel coil, due to the inflation pressure is balanced by the steel coil compression or extension stess given by:

Tk = 8 x Dm x F x K/ (pi x Dr³)    (eq. 9)

where K is a connector factor within the interval 1 <K <2 which is related to the ratio Dm/Dr.

The total elastic deformation (L) ol the spring i.e. steel coil in compression or in tension with a force (F) is given by:

L = 8 x Dm³ x Np x F/ G x Dr⁴    (eq. 10)

Combining eq (9) and (10) the stress of the coil is given by:

Tk = G x Dr x F x K/pi x Dm² x Np    (eq. 11)

F = applied force to the fluid (N)

Tk = shear modulus of the steel coil (N/mm²)

Dr = diameter of the steel rod (mm)

Dm = middle diameter of the steel coil (mm)

Di = internal diameter of the steel coil (mm)

G = shear modulus of the material (N/mm²)

L = elastic deformation length of the steel coil (mm)

Np = number of Working pitch

Taking into consideration eq (10) it is quite clear that in case of Np = 0 there will be no elastic deformation of steel coil. The steel coil will work as a compact steel rod, and no inflation will be possible at the extension section 3 in Fig. 1.

By regulating the Np value with the regulating device 10 in Fig. 1, one obtains the pre-set elastic deformation (L) of the coil which results in a pre-set balloon diameter at inflated condition.

In Fig. 4 the inner-body of the dilatation catheter of two lumen constructions is called the inner-tube, in the present invention the inner-tube is made of a steel coil 31 with a pitch corresponding to the diameter of the steel rod or ribbon and an outer diameter varying from 0.50 to 0.90 mm. A polymeric material 23 formed a thin film jacket of few microns on the outer surface of the steel coil 31. The polymeric material could be any type of thermoset or thermoplastic, in the present embodiment TEFLON (Du Pont trademark for polytetrafluorethylene) and cyanoacrylate were used. The steel coil 31 with its polymeric film jacket 23 forms the inner-body 5 of the dilatation catheter in Fig. 1. At the steel coil 31 proximal end a bushing 30 with three O-rings 26 and a threaded land 35 about half its length is made of metal or metal alloy like German silver and silver soldered at the unjacketed length of the inner-body 5. The bushing 30 takes house in the connector channel 61 thus forming a guiding cylinder for translational movement of the inner-body 5 regulated by the thumbwheel 20. Contiguous to the connector channel 61 a second guiding channel 62 coaxial to channel 61 of larger diameter than channel 61 with square shoulders 64 and 65 to channel 61 and channel 63 being of larger diameter than channel 62 houses the rotating inner-body of the thumbwheel 20.

The two channels 62 and 63 allow to accomodate the regulating device 10 which is encapsulated in the connector house 21 and supported by the connector shoulder 66. The channels 62 and 63 together with shoulder 66 form the outer casing of the regulating device 10. The channel 61, 62 and 63 are coaxial, thus providing a well controlled area of rotational movement of the thumbwheel 20. The shoulders 65 and 66 are intended to counterpose the applied force during regulating balloon diameter herein 66 and shoulder 65 during balloon inflatio.n. The thumbwheel translational movement during the two subsequent operations is limited by the tolerance of the housing which in this case is within 0.03 mm, substantially zero axial movement. In the center line of the thumbwheel a threaded channel is present to accomodate the threaded bushing 30 which is soldered to the inner-coil 31.

By clockwise rotation of the thumbwheel 20 a translational movement of the bushing 30 is obtained, and at the same time a corresponding translational movement of the "inner-tube" 5 anchored to the outer shaft 4 at the distal end 2 in Fig. 5, said inner-coil 31 is achieved. This translational movement due to the axial stiffness of the steel coil will result in a retraction action at the balloon section 3 in Fig. 1. The wide open mesh 34 of the reinforcing material 32 will be partially compressed by the clockwise rotation of the thumbwheel 20 thus giving rise to a pre-set balloon diameter. By changing sectionalwise the axial stiffness of the steel coil, the clockwise rotation of the thumbwheel 20 will pre-set the balloon diameter without compression of the open mesh 34; this means that when fluid pressure is applied at port 7 the expansion section will take up a balloon diameter of previously set value. The rotational movement of the regulating device 10 in Fig. 1 via the thumbwheel 20 upon the center axis of the connector 21, the bushing 30 proximal end will protrude from the thumbwheel guide wire port 51. In the bushing 30 proximal end an indicator 60 is embossed for the translational movement of the inner-coil 31. Together with the thumbwheel 20 circular indices not shown in the drawing form a slide gauge drive for balloon diameter reading.. When pressure is applied to the fluid at port 7 the sealing system made of a series of O-rings 26 prevents any possible passage of fluid or leakage at the channel 61. The outer tube 4 in Fig. 5 said shaft was built up of an inner-jacket 27 of elastomeric thermosetting material, silicone rubbers (Q) or thermoplastic polyurethane with a layer thickness varying between 50 and 100 microns. But other elastomeric materials like halogenes containing rubbers, i.e. fluorinated (F), brominated (Br), chlorinated (Cl), rubbers, acrylic, dienehomo and copolymers rubbers including thermoplastic rubber can be used as long as the physical and microbiological safety criterion is respected. The silicones, polyurethane and natural rubber of medical grade are well-known for similar applications. The diameter size of the entire longitudinal length outer tube 4 so-called shaft, has a very small diameter less than 4 French corresponding to 1.30 mm. The present invention in its basic principle does not prevent to manufacture dilatation catheters with shaft size less than 3 French, i.e. less than 1 mm. The limitation factors of size lower than 3 French are mostly found in the construction of micromechanical equipment.

The inner-jacket 27 together with liner 23 forms the intralumen 53 in Fig. 5 beginning at port 50 in the connector house at Fig. 5 and ending at the beginning of the tapered distal end 2 of the catheter. The inner-tube 6 together with the outer shaft 4 thus form the concentric channel for pressure application and consequently balloon inflation at the distal end 54 of the catheter. A reinforcing material of very high Joung modulus and extremely low elongation at break (E) less than 6% like steel or metals, glass, carbon, boron, aramid (Kevler (R) Du Pont trademark), ceramic, whiskers fibers, polymer alloys are suitable materials that can withstand high tension and compression force, consequently pressure load with almost no elongation or extremely low tension set. These materials have lower temperature dependence when compared to the usual reinforcing materials used today in balloon construction like polyester, polyamide or polyethylene high modulus. The former are less time temperature dependent than the latest, respectively, in dynamic or static application. The latest mentioned material suffers from elongation stability together with temperature dependence under stress application. The inner-layer or inner-jacket 27 does not allow to take up pressure above 0.1 atm. without showing dimension deformation at normal ambient temperature (RT). To prevent this physical instability a covering jacket, so-called "reinforcing materials", is necessary. The methods of reinforcing a polymeric tube are several, the most commonly used are: braiding, spiralling, knitting, pre-worked bandage lining.

In the present invention the braiding method was chosen and found easier than the pre-worked bandage lining. The inner-layer 27 is normally extruded on top of a flexible metal mandril which allows to be the stabilizing factor as far as dimension is concerned, during the reinforcement process.

The standard braiding machines are not suitable for this kind of tension sensitive operations. In the present invention a special operating dispensing tensioning rotating system "braider" had to be developed. A thermoset adhesive normally made of two chemical reacting components like the epoxy, cyano, isocyanates, silicones, and chloroprene rubber are suitable materials to be used as an adhesive film to be applied at the top layer of the liner 27, the liner to be chemically bonded to the reinforcing material 32 which is chemically pretreated to react with the adhesive on top of the liner 27.

In the present embodiment the above-mentioned fibers are all suitable as reinforcing material in a ribbon configuration or rods. The ribbon configuration is preferred due to lower catheter shaft diameter. The choice of the adhesive combined with the fiber type allows to manufacture shaft with different stiffness. The steel ribbon 0.05 x 0.10 mm was preferred due to higher longitudinal pushability and torqueability. The reinforcing material dispensed by the "braider" is positioned at a constant pitch 32 on top of the inner-layer 27. The outer surface of inner-layer 27 was treated with an adhesive film of 2-ethyl-cyanoacrylate during braiding in its almost total length except for the portion 54 constituting the balloon section, where the mesh of braiding are becoming more open 34. The angle (a) between the wound ribbon and the longitudinal axis is thus chosen to allow a controlled compression section covering the total length of the balloon. The controlled compression is obtained by operating the thumbwheel 20 in the connector. The outer layer 25 is made of an elastomeric material of very low modulus of elasticity at the balloon section, but other rubbers and thermoplastic materials like the block copolymer (AB), natural rubber (NR), polyisoprene (IR) and thermosetting polymeric materials with very low Joung modulus are suitable.

The silicones (Q) and polyurethane (Pu) were preferred in this embodiment. The remaining area of the "shaft" is mainly covered by a higher shore hardness polymer with thermosetting or thermoplastic properties. The outer layer is chemically bonded to the reinforcing material 32 by application of an adhesive with thermosetting or thermoplastic properties. In this embodiment the TPU shore hardness D 60 was used together with a 2-ethyl-cyanoacrylate as adhesive.

At the proximal end of the outer shaft 4 in Fig. 4 a plastic or metal bushing 33 is anchored to the reinforcing layer 32. The assembly is tightened to the connector parallel shoulders 37. A coded nut 24 makes a final smooth transition between connector shoulders 22 and outer shaft 4 proximal end. At the distal end 2 of the catheter in Fig. 5 the inner-tube 5 is sealed together with the outer shaft 4 thus allowing to establish an end closed channel for the fluid to be injected from port 7. The tapered section 2 in Fig. 5 provides a smooth and flexible transition to the very distal end of the catheter tip 1. The end tip of the catheter is a contiguous element of the inner-tube 5 with high flexibility like the distal tip of a soft guide wire, with an atraumatic configuration. This flexible tip 1 allows to track easy on the inserted guide wire which is normally used in dilatation procedure. The very distal end of the tip 1 being of high density metal like steel allows to be seen in the monitor thus providing the operating physician with a well defined position of the catheter tip. This is another innovative feature of the present invention which gives the physician a manoeuvrable device to be clearly seen in its total length when inserted in the human body and without contrast media being injected. The inner diameter 55 of the coated inner-coil 5 can vary from 0.3 -0.7 mm, the 0.7 mm lumen allows to house a guide wire of 0.52 mm (.020 inches).

To improve the visibility of the balloon section 54 through the monitor two gold markers 39 are equidistantly placed from the two shoulders of the balloon.

All the materials chosen for the present invention should be conform and suitable to the requirements prescribed by the U.S.Pharmacopea or European or the like. It is very clear that the present invention does allow to manufacture dilatation catheters with various material combinations not mentioned in the present disclosure, but still pertaining to the basic principle and scope of the invention. There is no doubt that the present invention has other applications than the above-mentioned always aiming for the benefit of the patient.

The descriptions of the accompanying drawings together with the following claims should be understood to cover all the singularities and the generic features which describe the idea and the scope of the present invention.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. A dilatation catheter to be used in percutaneous transluminal coronary angioplasty having an adjustable expandable section which under pressure takes up a pre-set balloon dimension to dilate stenotic coronary arteries according to their sizes without risk of under or over dilatation, the catheter comprising:
a flexible reinforced tubular body, said shaft, of inner and outer tubes made of elastomeric materials and reinforced by high Joung modulus material, steel, metals, or fibers of max. elongation at break less than 6% which lies tightly packed lor a major length and contiguous thereto at the distal end a more open packed shorter length, said expandable section, the catheter can be internally pressurized in its total length with or without radial expansion at the expansion section, said balloon, the radial expansion section of the shaft distal end is controlled by the thumbwheel placed in the connector house, together with a polymer coated steel coil forming the catheter internal lumen being able to take up compression and extension forces in y-x axis without substantial physical deformation, the adjustable radial expandable section of the shaft allows to be compressed in the longitudinal axis, thus achieving the pre-set ballon diameter which under pressure takes up a well defined expanded diameter,increasing or decreasing balloon diameter, can be achieved at equal internal pressure.

2. The dilatation catheter of claim 1 wherein the longitudinal flexible, tubular body is made of biocompatible rubbery materials, preferable polyurethane (PU), silicone rubber (Q) or other elastomers reinforced by braided steel ribbon or rod of closed and open mesh chemically and physically bonded to adjacent inner and outer polymer tubes.

3. The dilatation catheter of claim 2 wherein the reinforcing braided material is any of the metals, carbon, glass, boron, aramid, ceramic, whiskers fibers.

4. The dilatation catheter of claim 2 in which the longitudinal tubular body can be internally pressurized without radial expansion at the balloon section and has in its total length a uniform substantially constant circular size in a pressurized or unpressurized state.

5. The dilatation catheter of claim 2 hwerein the distal end of the longitudinal tubular body, said shaft, has an adjustable inflatable section, said balloon, the diameter of which can be regulated by the thumbwheel device in the connector house.

6. The dilatation catheter of claim 4, wherein the longitudinal tubular body a distal end inflatable section, said balloon, the diameter of which can increase or decrease with or without constant internal fluid pressure by the regulating thumbwheel device in the connector house.

7. The dilatation catheter of claim 1 further comprises an inner-body made of tight wound steel coil, polymeric coated on the outer surface forming a flexible and incompressible inner-tube.

8. The dilatation catheter of claim 7 wherein at the proximal end ot the inner-coil a threaded and sealing bushing device is soldered.

9. The dilatation catheter of claim 6 wherein the total length of the inner-coil is radiopaque, including distal tip.

10. The dilatation catheter of claim 1 further comprises: a connector to which the longitudinal tubular body and the inner-coil are sealed to two separated connecting ports, respectively.

11. The dilatation catheter of claim 10 further comprises a third port for pressure, time and temperature recording of the fluid.

12. The dilatation catheter of claim 1 further comprises an assembly of coaxial system wherein the adjacent surfaces of the inner-coil and the longitudinal tubular body are movable to each other at the confined expansion section, said balloon, during inflation and deflation.

13. The dilatation catheter of claim 12 wherein the confined pre-set expansion section is not coaxial movable during balloon inflation or deflation.

14. The dilatation catheter of claim 12 wherein the space in between allows the fluid to be injected and pressurized up to 30 atm. at temperature up to 80 degree C.

15. The dilatation catheter of claim 1 wherein the axial stiffness is adjustable by fluid pressure application.

16. The dilatation catheter of claim 1 wherein the operating physician does not need to change for other size balloon catheters during dilatation.

17. The dilatation catheter of claim 1 wherein the pre-set balloon diameter is controlled by the longitudinal axial movement of inner-coil through the thumbwheel rotating system where a slide gauge is incorporated thus to read its value.

18. The dilatation catheter of claim 1 is to provide a surgical instrument to dilate under full control of balloon diameter narrow stenotic strictures in the coronary blood vessel permitting to form together with a guide wire and guiding catheter a complete working assembly for dilatation purposes with minimum risk of balloon rupture, with no kinking possibilities while in use and preserving tracking capability and pushability.

# FIG.1

EP 0 388 486 A1

# FIG.2

# FIG.3

FIG. 2a

FIG. 3a

FIG. 2b

FIG. 3b

FIG. 2c

FIG. 3c

FIG.4

FIG.5

EP 0 388 486 A1

FIG 6

European Patent Office

**EUROPEAN SEARCH REPORT**

Application Number

EP 89 10 4959

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 186 267 (COOK INC.) | | |
| | | | **TECHNICAL FIELDS SEARCHED (Int. Cl.5)** |
| | | | A 61 M 29/02 A 61 M |

The present search report has been drawn up for all claims

| Place of search | Date of completion of the search | Examiner |
|---|---|---|
| THE HAGUE | 09-11-1989 | MIR Y GUILLEN V. |

EPO FORM 1503 03.82 (P0401)